(19) Europäisches Patentamt / European Patent Office / Office européen des brevets

(11) **EP 4 729 503 A1**

(12) **EUROPEAN PATENT APPLICATION**
published in accordance with Art. 153(4) EPC

(43) Date of publication:
**22.04.2026 Bulletin 2026/17**

(21) Application number: **24825902.0**

(22) Date of filing: **18.06.2024**

(51) International Patent Classification (IPC):
**C07C 17/386** (2006.01) **C07C 19/08** (2006.01)
**C09K 5/04** (2006.01)

(52) Cooperative Patent Classification (CPC):
(C-Sets available)
**C07C 17/386; C09K 5/045** (Cont.)

(86) International application number:
**PCT/JP2024/022124**

(87) International publication number:
**WO 2024/262501 (26.12.2024 Gazette 2024/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.06.2023 JP 2023100044**

(71) Applicant: **Daikin Industries, Ltd.**
**Osaka-shi, Osaka 530-0001 (JP)**

(72) Inventors:
• **HAGITA, Satoru**
  **Osaka-Shi, Osaka 530-0001 (JP)**
• **KAGAWA, Michiru**
  **Osaka-Shi, Osaka 530-0001 (JP)**
• **YANO, Hiroyuki**
  **Osaka-Shi, Osaka 530-0001 (JP)**
• **NOGUCHI, Atsushi**
  **Osaka-Shi, Osaka 530-0001 (JP)**

(74) Representative: **Hoffmann Eitle**
**Patent- und Rechtsanwälte PartmbB**
**Arabellastraße 30**
**81925 München (DE)**

(54) **METHOD FOR PRODUCING PURIFIED DIFLUOROMETHANE (HFC-32), AND COMPOSITION CONTAINING HFC-32**

(57) This disclosure provides a method for efficiently purifying difluoromethane (HFC-32).

Specifically, the present disclosure provides a method for producing a purified HFC-32, the method including: an extractive distillation step of contacting a composition containing difluoromethane (HFC-32) and a component X with a solvent A to obtain a composition containing a reduced component X from the composition,
wherein the component X is at least one selected from the group consisting of pentafluoroethane (HFC-125), 1,1,1-trifluoroethane (HFC-143a), and dichlorodifluoromethane (CFC-12), and
wherein the solvent A is at least one of an amine and a fluorinated ether.

Fig. 1

**(Cont. next page)**

(52) Cooperative Patent Classification (CPC): (Cont.)

C-Sets
**C07C 17/386, C07C 19/08**

## Description

Technical Field

The present disclosure relates to a method for producing purified difluoromethane (HFC-32) and a composition containing the HFC-32.

Background Art

HFC-32 is useful as a refrigerant, or a raw material of a mixed refrigerant combined with another component (for example, HFC-410A that is a mixed refrigerant of HFC-32 and HFC-125).
In reference to the present disclosure, for example, Patent Literature (PTL) 1 discloses a method for separating HFC-32 from HFC-125. More specifically, PTL 1 discloses "a method for separating HFC-32 from HFC-125 from a first mixture by using an extracting agent containing methylene chloride, the method comprising the steps of: adding the extracting agent to the first mixture to generate a second mixture; and separating HFC-32 from HFC-125 in the second mixture by extractive distillation the second mixture in an extractive distillation zone of a distillation column and thereby recovering HFC-125 as a column top product of the column and HFC-32 from a column bottom".

Citation List

Patent Literature

PTL 1: Japanese Patent Laid-Open No. 2007-91762

Summary of Invention

Technical Problem

An object of the present disclosure is to provide a method for efficiently purifying HFC-32, and a composition containing the HFC-32.

Solution to Problem

The present disclosure encompasses, for example, the embodiments described in the following Items.
Item 1. A method for producing a purified HFC-32, the method including:

an extractive distillation step of contacting a composition containing difluoromethane (HFC-32) and a component X with a solvent A to obtain a composition containing a reduced component X from the composition,
wherein the component X is at least one selected from the group consisting of pentafluoroethane (HFC-125), 1,1,1-trifluoroethane (HFC-143a), and dichlorodifluoromethane (CFC-12), and
the solvent A is at least one of an amine and a fluorinated ether.

Item 2. The method according to Item 1, further including:
a distillation step of distilling a composition containing the component X and the solvent A obtained from a column bottom of an extractive distillation column via the extractive distillation step to separate the component X from the solvent A.
Item 3. The method according to Item 1 or 2,
wherein the extractive distillation step using a first distillation column for performing the extractive distillation step is performed under a pressure of 0.05 to 5 MPaG (gauge pressure).
Item 4. The method according to Item 2 or 3,
wherein the distillation step using a second distillation column for performing the distillation step is performed under a pressure of 0.05 to 3 MPaG (gauge pressure).
Item 5. The method according to any one of Items 1 to 4, further including:
a solvent recovery step of recovering the solvent A used in the extractive distillation step to recycle the recovered solvent A in the extractive distillation step.
Item 6. The method according to any one of Items 1 to 5,

wherein the amine is represented by General Formula: $NR^1R^2R^3$,
where $R^1$, $R^2$, and $R^3$ are the same or different from each other, and represent hydrogen or a hydrocarbon group having 1 to 3 carbon atoms and optionally a substituent, with the proviso that a case where all of $R^1$, $R^2$, and $R^3$ are hydrogen is excluded.

Item 7. The method according to any one of Items 1 to 6, wherein the amine is at least one selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

Item 8. The method according to any one of Items 1 to 7,

wherein the fluorinated ether is represented by General Formula: $R^4$-O-$R^5$,
where $R^4$ and $R^5$ are the same as or different from each other, and represent $C_nH_mF_l$, where n is an integer of 1 to 20, m + l = 2n + 1, and m and l are an integer of 0 or more.

Item 9. The method according to any one of Items 1 to 8, wherein the fluorinated ether is at least one selected from the group consisting of 1,1,1,2,2,3,3,4,4-nonafluoro-4-methoxybutane (HFE-7100), 1,1,1,2,2,3,3,4,4-nonafluoro-4-ethoxybutane (HFE-7200), and 1,1,1,2,2,3,4,5,5,5-decafluoro-3-methoxy-4-(trifluoromethyl)pentane (HFE-7300).

Item 10. A composition, including:

difluoromethane (HFC-32);
a component X; and
a substance A,
wherein a total concentration of the three components is 99.5% by mass or more relative to a total of the composition,
the component X is at least one selected from the group consisting of pentafluoroethane (HFC-125), 1,1,1-trifluoroethane (HFC-143a), and dichlorodifluoromethane (CFC-12),
the substance A is at least one of an amine and a fluorinated ether, and
a content of the component X is more than 0% by mass and 0.4% by mass or less relative to the total of the composition, and a content of the substance A is more than 0% by mass and 0.1% by mass or less relative to the total of the composition.

Item 11. A composition, including:

difluoromethane (HFC-32); and
a substance A,
wherein a total concentration of the two components is 99.5% by mass or more relative to a total of the composition,
the substance A is at least one of an amine and a fluorinated ether, and
a content of the substance A is more than 0% by mass and 0.1% by mass or less relative to a total of the composition.

Item 12. A composition, including:

difluoromethane (HFC-32); and
a component X,
wherein a total concentration of the two components is 99.5% by mass or more relative to a total of the composition,
the component X is at least one selected from the group consisting of pentafluoroethane (HFC-125), 1,1,1-trifluoroethane (HFC-143a), and dichlorodifluoromethane (CFC-12), and
a content of the component X is more than 0% by mass and 0.4% by mass or less relative to a total of the composition.

Item 13. A method for producing a purified HFC-32, the method including:

an extractive distillation step of contacting a composition containing difluoromethane (HFC-32) and a component X with a solvent A to obtain a composition containing a reduced component X from the composition,
wherein the component X is at least one selected from the group consisting of pentafluoroethane (HFC-125), 1,1,1-trifluoroethane (HFC-143a), and dichlorodifluoromethane (CFC-12),
the solvent A is at least one of an amine and a fluorinated ether,
the extractive distillation step using a first distillation column for performing the extractive distillation step is performed under a pressure of 0.05 to 5 MPaG (gauge pressure),
the distillation step using a second distillation column for performing the distillation step is performed under a pressure of 0.05 to 3 MPaG (gauge pressure),

the amine is at least one selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine, and

the fluorinated ether is at least one selected from the group consisting of 1,1,1,2,2,3,3,4,4-nonafluoro-4-methoxybutane (HFE-7100), 1,1,1,2,2,3,3,4,4-nonafluoro-4-ethoxybutane (HFE-7200), and 1,1,1,2,2,3,4,5,5,5-decafluoro-3-methoxy-4-(trifluoromethyl)pentane (HFE-7300).

Advantageous Effects of Invention

According to the present disclosure, it is possible to efficiently purify HFC-32.

Brief Description of Drawings

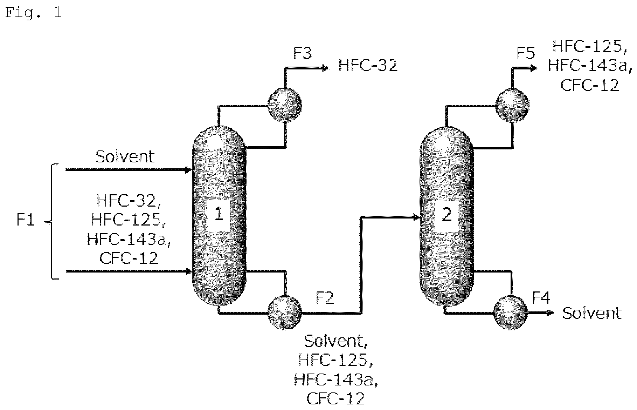

FIG. 1 is a view showing an overview of the process of a method for producing a purified HFC-32 in the present disclosure. In the figure, HFC-125, HFC-143a, and CFC-12 constituting the component X mean inclusion of at least one of the three components.

Description of Embodiments

As a result of diligent studies, the present inventors have found that HFC-32 can be efficiently purified by a production method including: an extractive distillation step of contacting, with a specific solvent, a composition containing HFC-32 and at least one (component X) selected from the group consisting of pentafluoroethane (HFC-125), 1,1,1-trifluoroethane (HFC-143a), and dichlorodifluoromethane (CFC-12), to obtain a composition containing a reduced component X from the composition.

The present disclosure has been completed as a result of further researches based on the above finding.

Herein, a numerical range represented using "to" means the range that includes the numerical values described before and after "to" as the lower limit and the upper limit (that is, "or more, or less").

Herein, the azeotropic composition means a composition that shows no difference in compositional features between a liquid phase and a gas phase under a constant pressure and behaves as if it were a single substance.

Herein, in the composition that can form azeotropic compositional features, a near-azeotropic composition is a composition having compositional features close to the azeotropic compositional features, and means compositional features exhibiting behaviors close to those of the azeotropic composition. The near-azeotropic composition can be distilled and/or reflexed with little change in the compositional features. Therefore, the near-azeotropic composition can be handled almost similarly to the azeotropic composition. Note that, one feature of the near-azeotropic composition is that a difference in pressure between the boiling point curve and the dew point curve in a diagram of pressure - compositional features is within 5%.

Herein, the standard boiling point means a boiling point at a standard atmospheric pressure of 1,013.25 hPa.

Herein, the gauge pressure means a relative pressure based on an atmospheric pressure, and means a pressure difference obtained by subtracting an atmospheric pressure from an absolute pressure. Herein, the gauge pressure is indicated by appending "G", for example, MPaG. On the other hand, when "G" is not appended, it is an absolute pressure.

Herein, the "purity" of a refrigerant means the ratio of a component (mole (mol)% or mass%) by the quantitative analysis of the gas chromatography.

Herein, the main component means a component contained in an amount of preferably 85 mol% to 99.9 mol%, more preferably 90 mol% to 99.9 mol%, still more preferably 95 mol% to 99.9 mol%, and particularly preferably 99 mol% to 99.9 mol%.

Herein, the extractive distillation means the following distillation operation. That is, an extraction solvent is added to a mixture that has a relative volatility of about 1 and contains two or three components whose standard boiling points are extremely close to each other and which are difficult to separate by ordinary distillation, or to a mixture obtained by combining components having azeotropic compositional features, to thereby obtain a mixture for extraction. As a result, the relative volatility of the original two or three components is far away from 1, facilitating separation. When the relative volatility is 1, separation by distillation is impossible.

Herein, regarding the relative volatility ($\alpha$), in the case where a composition containing at least a target component A and a target component B is in the gas-liquid equilibrium state, when a molar fraction of the liquid phase component A is $x_A$, a molar fraction of the liquid phase component B is $x_B$, a molar fraction of the gas phase component A that is in the equilibrium state with the liquid phase is $y_A$, and a molar fraction of the gas phase component B is $y_B$, the relative volatility of the component A to the component B is defined by $\alpha._{A \to B} = (y_A / x_A) / (y_B / x_B)$.

Herein, when the component A is HFC-32 and the component B is HFC-125, the relative volatility of the component A to the component B, "relative volatility of HFC-32 to HFC-125" is described as "$\alpha_{32 \to 125}$".

The present disclosure includes the following embodiments.

A production method of the present disclosure is a method for producing a purified HFC-32, the method including:

an extractive distillation step of contacting a composition containing difluoromethane (HFC-32) and a component X with a solvent A to obtain a composition containing a reduced component X from the composition, wherein the component X is at least one selected from the group consisting of pentafluoroethane (HFC-125), 1,1,1-trifluoroethane (HFC-143a), and dichlorodifluoromethane (CFC-12), and the solvent A is at least one of an amine and a fluorinated ether.

In the extractive distillation step, when a composition particularly containing HFC-32 and a component X (also referred to as "composition for extractive distillation" or "raw material composition") is an azeotropic composition or a near-azeotropic composition, the production method of the present disclosure is preferably applied. Here, all of HFC-125, HFC-143a, and CFC-12, which are the component X, can be an azeotropic composition or a near-azeotropic composition in the state of a mixture with HFC-32. In recent years, recovering used refrigerants (particularly mixed refrigerants) and recycling specific components have been promoted. Therefore, in the present disclosure, for example, used HFC-410A (a mixed refrigerant of HFC-32 and HFC-125) can be applied as the composition for extractive distillation (raw material composition).

(Extractive Distillation Step)

The extractive distillation step in the production method of the present disclosure is a step of contacting a composition (composition for extractive distillation) containing a component X (at least one selected from the group consisting of HFC-125, HFC-143a, and CFC-12) and HFC-32 with a solvent A (at least one of an amine and a fluorinated ether), to obtain a composition containing a reduced component X from the composition. In other words, the extractive distillation step is a step of subjecting a composition (composition for extractive distillation) containing the component X and HFC-32 to extractive distillation in the presence of the solvent A, to obtain a composition containing a reduced component X from the composition. Note that, in the present disclosure, the "reduced" in the extractive distillation step means that a content proportion of the specific compound (component X) in the composition for extractive distillation is reduced.

The composition for extractive distillation contains the component X and HFC-32 in a total of their concentrations of preferably 99.5% by mass or more, more preferably 99.7% by mass or more, still more preferably 99.8% by mass or more, and even still more preferably 99.9% by mass or more. As the composition for extractive distillation, when the main component of the component X is, for example, HFC-125, after used HFC-410A is recovered, a previous separation step (optional distillation step or the like) is performed according to the necessity, to remove a by-product that can be easily separated, and a composition (particularly, an azeotropic composition or a near-azeotropic composition) that preferably contains HFC-125 and HFC-32 in the total of their concentrations described above can be applied.

Similarly, via an optional distillation step etc., a composition for extractive distillation can be formed from the used recovered product of a mixed refrigerant containing, as constituents, HFC-32 and at least one component X selected from the group consisting of:

· HFC-407A(HFC-32+HFC-125+HFC-134a)
· HFC-407B(HFC-32+HFC-125+HFC-134a)
· HFC-407C(HFC-32+HFC-125+HFC-134a)
· HFC-407D(HFC-32+HFC-125+HFC-134a)
· HFC-407E(HFC-32+HFC-125+HFC-134a)
· HFC-407F(HFC-32+HFC-125+HFC-134a)
· HFC-407H(HFC-32+HFC-125+HFC-134a)
· HFC-407I(HFC-32+HFC-125+HFC-134a)
· HFC-410B(HFC-32+HFC-125)
· HFC-425A(HFC-32+HFC-134a+HFC-227ea)
· HFC-427A(HFC-32+HFC-125+HFC-134a+HFC-143a)
· R-438A(HFC-32+HFC-125+HFC-134a+R-600+R601a)
· R-439A(HFC-32+HFC-125+R600a)
· R-442A(HFC-32+HFC-125+HFC-134a+HFC-152a+HFC-227ea)
· R-444A(HFC-32+HFC-152a+HFO-1234ze(E))
· R-448A(HFC-32+HFC-125+HFC-134a+HFO-1234ze(E)+HFO-1234yf)
· R-449A(HFC-32+HFC-125+HFC-134a+HFO-1234yf)
· R-452A(HFC-32+HFC-125+HFO-1234yf)

· HFC-458A(HFC-32+HFC-125+HFC-134a+HFC-227ea+HFC-236fa)
· R-463A(R-744+HFC-32+HFC-125+HFO-1234yf+HFC-134a)
· R-466A(HFC-32+HFC-125+CF$_3$I)
· R-504(HFC-32+HCFC-115)

The composition for extractive distillation preferably consists of only HFC-32 and the component X, but may contain inevitable impurities depending on the condition of the preparation process of the composition for extractive distillation. Note that, the phrase "the composition for extractive distillation contains the component X and HFC-32 in a total of their concentrations of preferably 99.5% by mass or more" described above means that the composition for extractive distillation substantially consists of only HFC-32 and the component X, or the composition for extractive distillation contains only inevitable impurities as another component even if it contains another component.

HFC-125 (standard boiling point: -48.1°C) that is one of the components X and HFC-32 (standard boiling point: -51.7°C) are azeotropic or near-azeotropic, and an azeotropic composition or near-azeotropic composition containing HFC-125 and HFC-32 has a boiling point of a temperature, which is lower than a boiling point of HFC-125 or HFC-32 (at 0.1013 MPa, the azeotropic compositional features are HFC-32 / HFC-125 = 87.8 / 12.2 (mol%), and a temperature of -51.8°C). HFC-143a (standard boiling point: -47.3°C) that is one of the components X and HFC-32 (standard boiling point: -51.7°C) are azeotropic or near-azeotropic, and an azeotropic composition or near-azeotropic composition containing HFC-143a and HFC-32 has a boiling point of a temperature, which is lower than a boiling point of HFC-143a or HFC-32 (at 0.1013 MPa, the azeotropic compositional features are HFC-32 / HFC-143a = 77.8 / 22.2 (mol%), and a temperature of -52.5°C). Moreover, CFC-12 (standard boiling point: -29.8°C) that is one of the components X and HFC-32 (standard boiling point: -51.7°C) are azeotropic or near-azeotropic, and an azeotropic composition or near-azeotropic composition containing CFC-12 and HFC-32 has a boiling point of a temperature, which is lower than a boiling point of CFC-12 or HFC-32 (at 0.1013 MPa, the azeotropic compositional features are HFC-32 / CFC-12 = 96.5 / 3.5 (mol%), and a temperature of -51.7°C). As the compositional features of the composition for extractive distillation supplied to the extractive distillation step, the molar ratio of HFC-32 is preferably 50.0% or more and 99.999% or less, and more preferably 70.0% or more and 99.999% or less. As the compositional features of the composition containing HFC-32 as a main component obtained by the extractive distillation, the molar ratio of HFC-32 is preferably 99.500% or more, more preferably 99.900% or more, and still more preferably 99.999% or more.

The extractive distillation step is preferably a step of contacting the composition for extractive distillation with the solvent A to perform the extractive distillation, thereby obtaining a composition that contains HFC-32 but does not substantially contain the component X.

Herein, "does not substantially contain the component X" means that the content of the component X in the composition obtained in the extractive distillation step (when two or more of HFC-125, HFC-143a, and CFC-12 are contained, the total of their components) is preferably less than 1% by mass, more preferably less than 0.5% by mass, and particularly preferably less than 0.1% by mass.

In the extractive distillation step, as the extraction solvent, the solvent A that is at least one of an amine and a fluorinated ether is used. The extraction solvent herein preferably consists of only at least one of an amine and a fluorinated ether, but may contain inevitable impurities within such a range that does not affect the extractive distillation step. The solvent A is also referred to simply as the extraction solvent in the present disclosure.

The amine may be a liquid amine that is the extraction solvent, and is preferably an amine represented by General Formula: $NR^1R^2R^3$

wherein $R^1$, $R^2$, and $R^3$ are the same as or different from each other, and represent hydrogen or a hydrocarbon group having 1 to 3 carbon atoms and optionally a substituent, with the proviso that a case where all of $R^1$, $R^2$, and $R^3$ are hydrogen is excluded.

The amine preferably has a standard boiling point of - 10 to 160°C.

The amine is preferably at least one selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine. Among these amines, at least one of diethylamine and triethylamine is more preferable because the operation conditions of a distillation column are easily adjusted to the pressure and temperature that are easily operated. The Cas Nos. and the standard boiling points of these amines are described in the following Table 1.

[Table 1]

| Substance Name | Cas No. | Standard Boiling Point (°C) |
|---|---|---|
| Monomethylamine | 74-89-5 | -6 |
| Dimethylamine | 124-40-3 | 7.0 |

(continued)

| Substance Name | Cas No. | Standard Boiling Point (°C) |
|---|---|---|
| Trimethylamine | 75-50-3 | 2.9 |
| Monoethylamine | 75-04-7 | 38 |
| Diethylamine | 109-89-7 | 56 |
| Triethylamine | 121-44-8 | 90 |
| Mono-n-propylamine | 107-10-8 | 48 |
| Di-n-propylamine | 142-84-7 | 110 |
| Tri-n-propylamine | 102-69-2 | 156 |
| Mono-isopropylamine | 75-31-0 | 32 |
| Di-isopropylamine | 108-18-9 | 84 |

These amines can be used alone or in combination with two kinds or more of amines.

The fluorinated ether may be an extraction solvent, but is preferably a fluorinated ether represented by General Formula $R^4$-O-$R^5$, where $R^4$ and $R^5$ are the same as or different from each other, and represent $C_nH_mF_l$, where n is an integer of 1 to 20, $m + l = 2n + 1$, and m and l are an integer of 0 or more.

The fluorinated ether is preferably at least one selected from the group consisting of 1,1,1,2,2,3,3,4,4-nonafluoro-4-methoxybutane (HFE-7100), 1,1,1,2,2,3,3,4,4-nonafluoro-4-ethoxybutane (HFE-7200), and 1,1,1,2,2,3,4,5,5,5-decafluoro-3-methoxy-4-(trifluoromethyl)pentane (HFE-7300). Among these fluorinated ethers, at least one selected from the group consisting of HFE-7100, HFE-7200, and HFE-7300 is particularly preferable from the viewpoints of the handling property, the operation temperature, and the like. The Cas Nos. and the standard boiling points of these fluorinated ethers are described in the following Table 2.

[Table 2]

| Substance Name | Cas No. | Standard Boiling Point (°C) |
|---|---|---|
| HFE-7100 | 163702-07-6<br>163702-08-7 | 61 |
| HFE-7200 | 163702-05-4<br>163702-06-5 | 76 |
| HFE-7300 | 132182-92-4 | 98 |

[Note that, because both HFE-7100 and HFE-7200 are a two-component mixture, two Cas Nos. of each constituent are described.]

These fluorinated ethers can be used alone or in combination with two kinds or more of fluorinated ethers.

A mixing ratio between the amine and the fluorinated ether in the solvent A is not limited. The solvent A may be the amine alone, may be the fluorinated ether alone, or may be a mixture of two kinds of these substances contained at any proportion.

Regarding the temperature range of the standard boiling point in the above extractive distillation step, the extraction solvent and the compound to be separated in the extractive distillation step may have a temperature difference sufficient to allow separation by simple distillation, stripping, etc., usually may have a temperature difference of 20°C or more. However, if the standard boiling point is too high, the extractive solvent itself may be decomposed. Therefore, from the viewpoint of efficiently performing the extractive distillation, the standard boiling point of the extraction solvent is preferably 30 to 135°C, more preferably 35 to 120°C, even more preferably 40 to 100°C, and particularly preferably 50 to 90°C.

The amount of the extraction solvent used in the extractive distillation step is preferably 1 time molar equivalent or more and 30 times molar equivalents or less, and more preferably 5 times molar equivalents or more and 25 times molar equivalents or less, relative to the composition for extractive distillation supplied to the extractive distillation column.

The concentration of the component X in the composition for extractive distillation in the extractive distillation step is preferably 50 mol% or less, more preferably 30 mol% or less, and still more preferably 10 mol% or less.

The number of theoretical plates of the extractive distillation column used in the extractive distillation step is preferably 10 or more, and more preferably 20 or more. In addition, the number of theoretical plates of the extractive distillation column used in the extractive distillation step is preferably 100 or less, and more preferably 70 or less, from an economical viewpoint.

In the extractive distillation step, the extraction solvent is preferably supplied to the upper row of the extractive distillation column. The extraction solvent used in the extractive distillation step is recovered in the extraction solvent recovery step described later, and is preferably a recycled extraction solvent.

In the extractive distillation step, the pressure for performing the extractive distillation (the pressure of the first distillation column) is preferably 0.05 to 5 MPaG (gauge pressure). The lower limit of the pressure is preferably 0.05 MPaG, more preferably 0.1 MPaG, still more preferably 0.25 MPaG, and particularly preferably 0.5 MPaG. The upper limit of the pressure is preferably 5 MPaG, more preferably 4 MPaG, still more preferably 3 MPaG, and particularly preferably 2 MPaG.

The extractive distillation step can be performed through discontinuous operation or continuous operation, and is preferably performed through continuous operation from an industrial viewpoint. Moreover, when the extractive distillation is repeated, a distilled component can have a higher purity.

In the extractive distillation step, in the case where HFC-32 is distilled from the composition for extractive distillation, when the extraction solvent is added, such an extraction solvent that the relative volatility of HFC-32 to the component X is allowed to become preferably 1.70 or more and more preferably 2.00 or more is preferably used. As a result, when the gas phase molar fraction of HFC-32 increases, HFC-32 increases in the gas phase, HFC-32 can be separated from the column top of the extractive distillation column, and the extraction solvent and the component X can be obtained from the column bottom of the extractive distillation column.

(Extraction Solvent Recovery Step)

The production method of the present disclosure preferably includes an extraction solvent recovery step of recovering the extraction solvent used in the extractive distillation step, and recycling the recovered extraction solvent in the extractive distillation step.

The extraction solvent recovery step includes a distillation step of distilling a composition containing the component X and the solvent A obtained from the column bottom of the extractive distillation column via the extractive distillation step to separate the component X from the solvent A (hereinafter, also referred to as "distillation step"), which can be performed by recovering the extraction solvent from the composition containing the solvent A and recycling it in the extractive distillation step. Note that, the distillation step of separating the component X from the solvent A is preferably a distillation step of separating a composition containing the component X as a main component from a composition containing the extraction solvent as a main component.

The number of theoretical plates of the solvent recovering column (second distillation column) used in the distillation step is preferably 5 or more, and more preferably 10 or more. In addition, the number of theoretical plates of the solvent recovering column is preferably 40 or less, and more preferably 30 or less, from an economical viewpoint.

In the distillation step, the pressure for performing the distillation is preferably 0.05 to 3 MPaG (gauge pressure). The lower limit of the pressure is preferably 0.05 MPaG, and more preferably 0.1 MPaG. The upper limit of the pressure is preferably 3 MPaG, and more preferably 2.5 MPaG.

In the distillation step, a composition containing the component X as a main component can be separated from the column top of the solvent recovering column, and the composition containing the extraction solvent as a main component can be obtained from the column bottom of the solvent recovering column.

The extraction solvent recovery step can be performed by recovering the extraction solvent from the composition containing the extraction solvent as a main component obtained from the column bottom in the distillation step, and recycling the extraction solvent in the extractive distillation step. Note that, the extraction solvent recovered from the column bottom can be further subjected to an optional separation step such as rectification according to the necessity in order to remove impurities before the extraction solvent is recycled in the extractive distillation step.

The distillation step can be performed through discontinuous operation or continuous operation, and is preferably performed through continuous operation from an industrial viewpoint.

The production method of the present disclosure preferably includes the extractive distillation step and the extraction solvent recovery step, and more preferably includes an optional previous distillation step, an extractive distillation step, and an extraction solvent recovery step, in order to increase the purity of the composition for extractive distillation.

(Composition Containing HFC-32, Component X, And Substance A (Refrigerant 1))

The present disclosure encompasses a composition containing difluoromethane (HFC-32), a component X, and a substance A (hereinafter, also referred to as "refrigerant 1"). The component X, which is the same as that in the item of the extractive distillation step, is at least one selected from the group consisting of pentafluoroethane (HFC-125), 1,1,1-trifluoroethane (HFC-143a), and dichlorodifluoromethane (CFC-12). In addition, the kind of substance A, which is the same as that of the solvent A described in the item of the extractive distillation step, is at least one of an amine and a fluorinated ether.

The refrigerant 1 is a composition containing HFC-32, the component X, and the substance A. A total concentration of the three components is 99.5% by mass or more relative to a total of the composition, a content of the component X is more than 0% by mass and 0.4% by mass or less relative to a total of the composition, and a content of the substance A is more than 0% by mass and 0.1% by mass or less relative to the total of the composition. The refrigerant 1 is a composition in which trace amounts of the component X and the extraction solvent (solvent A) remain, in the composition containing a purified HFC-32 finally obtained by the production method of the present disclosure. Note that, the description of "more than 0% by mass" can be described as "0.01% by mass or more".

The total concentration of the three components in the refrigerant 1 is preferably 99.7% by mass or more, more preferably 99.8% by mass or more, and still more preferably 99.9% by mass or more, relative to a total of the composition.

The refrigerant 1 particularly preferably consists of only HFC-32, the component X, and the substance A, but may contain inevitable impurities.

The application of the refrigerant 1 is not limited, but the refrigerant 1 can be applied to the applications such as a refrigerant for air conditioning, a heat transfer medium, and a refrigerant for car air conditioner.

(Composition Containing HFC-32 And Component X (Refrigerant 2))

The present disclosure encompasses a composition containing difluoromethane (HFC-32) and the component X (also referred to as "refrigerant 2"). Note that, the kind of component X is the same as the kind described above.

The refrigerant 2 is a composition containing HFC-32 and the component X. A total concentration of the two components is 99.5% by mass or more relative to a total of the composition, and a content of the component X is more than 0% by mass and 0.4% by mass or less relative to a total of the composition. The refrigerant 2 is a composition in which a trace amount of the component X remains, in the composition containing a purified HFC-32 finally obtained by the production method of the present disclosure. Note that, the description of "more than 0% by mass" can be described as "0.01% by mass or more".

The total concentration of the two components in the refrigerant 2 is preferably 99.7% by mass or more, more preferably 99.8% by mass or more, and still more preferably 99.9% by mass or more, relative to the total of the composition.

The refrigerant 2 particularly preferably consists of only HFC-32 and the component X, but may contain inevitable impurities.

The application of the refrigerant 2 is not limited, but the refrigerant 2 can be applied to the applications such as a refrigerant for air conditioning, a heat transfer medium, and a refrigerant for car air conditioner.

(Composition Containing Purified HFC-32)

The present disclosure encompasses a composition containing HFC-32 and a substance A as a composition containing a purified HFC-32 (also referred to as "composition 1"). Note that, the kind of substance A is the same as the kind described above.

The composition 1 is a composition containing HFC-32 and the substance A. A total concentration of the two components is 99.5% by mass or more relative to a total of the composition, and a content of the substance A is more than 0% by mass and 0.1% by mass or less relative to the total of the composition. The composition 1 is a composition in which a trace amount of the extraction solvent (solvent A) remains, in the composition containing a purified HFC-32 finally obtained by the production method of the present disclosure. Note that, the description of "more than 0% by mass" can be described as "0.01% by mass or more".

The total concentration of the two components in the composition 1 is preferably 99.7% by mass or more, more preferably 99.8% by mass or more, and still more preferably 99.9% by mass or more, relative to the composition 1.

The composition 1 particularly preferably consists of only HFC-32 and the substance A, but may contain inevitable impurities.

The application of the composition 1 is not limited, but the composition 1 can be applied to the applications such as a leak detection agent, a stabilizer, and a tracer when a refrigerant is used.

Examples

Examples will be described below and the present disclosure will be specifically explained. Note that, the present disclosure shall not be limited to these Examples.

In Examples, the concentration of each component such as HFC-32 was measured using the following measurement apparatus under the following measurement conditions.

Measurement apparatus: Gas chromatography (FID detector was used)

Method for calculating the concentration of each component:

-

Concentration of HFC-32 = the number of moles of HFC-32 / (the number of moles of HFC-32 + the number of moles of component X)

-

Concentration of component X = the number of moles of component X / (the number of moles of HFC-32 + the number of moles of component X)

Examples 1 to 5 and Comparative Examples 1 to 14

Six times by mole of each solvent was added to a composition containing 70 mol% of HFC-32 and 30 mol% of HFC-125, and a relative volatility $\alpha_{32\rightarrow125}$ of HFC-32 to HFC-125 was measured at 5°C.

In Example 1, diethylamine was used as a solvent, and the relative volatility $\alpha_{32\rightarrow125}$ was 2.53. In Example 2, triethylamine was used as a solvent, and the relative volatility $\alpha_{32\rightarrow125}$ was 2.08. In Example 3, HFE-7300 was used as a solvent, and the relative volatility $\alpha_{32\rightarrow125}$ was 2.00. In Example 4, HFE-7200 was used as a solvent, and the relative volatility $\alpha_{32\rightarrow125}$ was 1.77. In Example 5, HFE-7100 was used as a solvent, and the relative volatility $\alpha_{32\rightarrow125}$ was 1.73. In Comparative Example 1, isopropyl alcohol (IPA) was used as a solvent, and the relative volatility $\alpha_{32\rightarrow125}$ was 1.66. In Comparative Example 2, tetrahydrofuran (THF) was used as a solvent, and the relative volatility $\alpha_{32\rightarrow125}$ was 1.49. In Comparative Example 3, ethanol was used as a solvent, and the relative volatility $\alpha_{32\rightarrow125}$ was 1.48. In Comparative Example 4, 2-butanol was used as a solvent, and the relative volatility $\alpha_{32\rightarrow125}$ was 1.43. In Comparative Example 5, methyl ethyl ketone (MEK) was used as a solvent, and the relative volatility $\alpha_{32\rightarrow125}$ was 1.43. In Comparative Example 6, n-propanol was used as a solvent, and the relative volatility $\alpha_{32\rightarrow125}$ was 1.40. In Comparative Example 7, ethyl acetate was used as a solvent, and the relative volatility $\alpha_{32\rightarrow125}$ was 1.40. In Comparative Example 8, isobutanol was used as a solvent, and the relative volatility $\alpha_{32\rightarrow125}$ was 1.32. In Comparative Example 9, methanol was used as a solvent, and the relative volatility $\alpha_{32\rightarrow125}$ was 1.26. In Comparative Example 10, acetone was used as a solvent, and the relative volatility $\alpha_{32\rightarrow125}$ was 1.24. In Comparative Example 11, hexane was used as a solvent, and the relative volatility $\alpha_{32\rightarrow125}$ was 1.18. In Comparative Example 12, 5-fluoropropanol was used as a solvent, and the relative volatility $\alpha_{32\rightarrow125}$ was 0.92. In Comparative Example 13, trifluoroethanol was used as a solvent, and the relative volatility $\alpha_{32\rightarrow125}$ was 0.74. In Comparative Example 14, acetonitrile was used as a solvent, and the relative volatility $\alpha_{32\rightarrow125}$ was 0.69.

The results are summarized in the following Table 3.

[Table 3]

| | Solvent | Relative Volatility $\alpha_{32\rightarrow125}$ |
|---|---|---|
| Example 1 | Diethylamine | 2.53 |
| Example 2 | Triethylamine | 2.08 |
| Example 3 | HFE-7300 | 2.00 |
| Example 4 | HFE-7200 | 1.77 |
| Example 5 | HFE-7100 | 1.73 |
| Comparative Example 1 | IPA | 1.66 |
| Comparative Example 2 | THF | 1.49 |
| Comparative Example 3 | Ethanol | 1.48 |
| Comparative Example 4 | 2-Butanol | 1.43 |
| Comparative Example 5 | MEK | 1.43 |
| Comparative Example 6 | n-Propanol | 1.40 |
| Comparative Example 7 | Ethyl acetate | 1.40 |

(continued)

|  | Solvent | Relative Volatility $\alpha_{32\to125}$ |
|---|---|---|
| Comparative Example 8 | Isobutanol | 1.32 |
| Comparative Example 9 | Methanol | 1.26 |
| Comparative Example 10 | Acetone | 1.24 |
| Comparative Example 11 | Hexane | 1.18 |
| Comparative Example 12 | 5-Fluoropropanol | 0.92 |
| Comparative Example 13 | Trifluoroethanol | 0.74 |
| Comparative Example 14 | Acetonitrile | 0.69 |

The above results demonstrated that amines such as diethylamine and triethylamine, and fluorinated ethers such as HFE-7300, HFE-7200, and HFE-7100 are effective as an extraction solvent.

It is found that both the relative volatility $\alpha_{32\to143a}$ of HFC-32 to HFC-143a at 5°C and the relative volatility $\alpha_{32\to12}$ of HFC-32 to CFC-12 at 5°C are 1.70 or more in each of the solvents used in Examples 1 to 5, and these solvents are effective not only in the separation of HFC-32 from HFC-125 by extractive distillation but also in the separation of HFC-32 from HFC-143a and/or CFC-12 by extractive distillation.

Example 6

Based on the flow diagram shown in FIG. 1, the composition for extractive distillation containing HFC-32 and the component X (HFC-125) was used as a raw material, HFE-7100 was used as an extraction solvent, and purified HFC-32 was produced by a process including the extractive distillation step and the extraction solvent recovery step.

(Extractive Distillation Step)

The composition for extractive distillation was supplied to the extractive distillation column at a flow rate of 956 mol/hr from the 45th plate from the column top of the extractive distillation column with the number of theoretical plates of 70. The extraction solvent (HFE-7100) was supplied at a flow rate of 9650 mol/hr from the third plate of the extractive distillation column. The operation pressure of the extractive distillation column was 0.5 MPaG, and the temperature of the column top was -10°C.

(Extraction Solvent Recovery Step)

The bottoms withdrawn from the column bottom in the extractive distillation step were fed from the fifth plate from the column top of the extraction solvent recovering column with the number of theoretical plates of 15 (hereinafter, referred to as "solvent recovering column"), and HFC-125 having a purity of 99.99% was recovered from the column top. The operation pressure of the solvent recovering column was 0.4 MPaG, and the temperature of the column top was -9°C.

The bottoms containing HFE-7100 withdrawn from the column bottom of the recovering column (flow rate of HFE-7100: 144 mol/hr) were recycled and used in the extractive distillation step.

The mass balance of Example 6 using the process in FIG. 1 was as follows: HFC-32: 667.1 mol/hr, HFC-125: 289.1 mol/hr, and HFE-7100 (extraction solvent): 9650.6 mol/hr in F1. The mass balance in F2 was as follows: HFC-32: 6.7 mol/hr, HFC-125: 289.1 mol/hr, and HFE-7100 (extraction solvent): 9650.2 mol/hr. The mass balance in F3 was as follows: HFC-32: 660.4 mol/hr, HFC-125: 0.0014 mol/hr, and HFE-7100 (extraction solvent): trace amount (0.1 ppm or less). The mass balance in F4 was as follows: HFC-32: trace amount (0.1 ppm or less), HFC-125: 0.005 mol/hr, and HFE-7100 (extraction solvent): 9650.6 mol/hr. The mass balance in F5 was as follows: HFC-32: 6.7 mol/hr, HFC-125: 289.1 mol/hr, and HFE-7100 (extraction solvent): 0.002 mol/hr. The following Table 4 shows the breakdown of the contents of the three components at positions F1, F2, F3, F4, and F5 in the process of Example 6 (the total content of the three components is 100 mol%).

[Table 4]

|  | F1 | F2 | F3 | F4 | F5 |
|---|---|---|---|---|---|
| HFC-32 | 6.3% | 0.067% | 99.9998% | 0.1 ppm or less | 2.255% |

(continued)

|  | F1 | F2 | F3 | F4 | F5 |
|---|---|---|---|---|---|
| HFC-125 | 2.7% | 2.907% | 0.0002% | 0.0001% | 97.744% |
| HFE7100 | 91.0% | 97.026% | 0.1 ppm or less | 99.9999% | 0.1 ppm or less |
| * The unit is mol%. | | | | | |

The amines shown in Examples 1 and 2 and the fluorinated ethers shown in Examples 3 to 5 have a relative volatility $\alpha_{32 \rightarrow \text{component X}}$ of HFC-32 to the component X (HFC-125, HFC-143a, and CFC-12) of 1.70 or more, which is larger than that of each Comparative Example (1.69 or less), and exhibit an improved separation efficiency compared to that of each Comparative Example because the concentration of HFC-32 in the gas phase can be relatively highly ensured. Therefore, for the separation of the component X from HFC-32, the process of the present disclosure that uses at least one extraction solvent of an amine and a fluorinated ether (solvent A) in the extractive distillation step is very effective.

Reference Signs List

1. Extractive distillation column
2. Solvent recovering column

## Claims

1. A method for producing a purified HFC-32, the method comprising:

   an extractive distillation step of contacting a composition containing difluoromethane (HFC-32) and a component X with a solvent A to obtain a composition containing a reduced component X from the composition,
   wherein the component X is at least one selected from the group consisting of pentafluoroethane (HFC-125), 1,1,1-trifluoroethane (HFC-143a), and dichlorodifluoromethane (CFC-12), and
   the solvent A is at least one of an amine and a fluorinated ether.

2. The method according to claim 1, further comprising:
   distillation step of distilling a composition containing the component X and the solvent A obtained from a column bottom of an extractive distillation column via the extractive distillation step to separate the component X from the solvent A.

3. The method according to claim 1 or 2,
   wherein the extractive distillation step using a first distillation column for performing the extractive distillation step is performed under a pressure of 0.05 to 5 MPaG (gauge pressure).

4. The method according to claim 2 or 3,
   wherein the distillation step using a second distillation column for performing the distillation step is performed under a pressure of 0.05 to 3 MPaG (gauge pressure).

5. The method according to any one of claims 1 to 4, further comprising:
   solvent recovery step of recovering the solvent A used in the extractive distillation step to recycle the recovered solvent A in the extractive distillation step.

6. The method according to any one of claims 1 to 5,

   wherein the amine is represented by General Formula: $NR^1R^2R^3$,
   where $R^1$, $R^2$, and $R^3$ are the same as or different from each other, and represent hydrogen or a hydrocarbon group having 1 to 3 carbon atoms and optionally a substituent, with the proviso that a case where all of $R^1$, $R^2$, and $R^3$ are hydrogen is excluded.

7. The method according to any one of claims 1 to 6,

wherein the amine is at least one selected from the group consisting of monomethylamine, dimethylamine, trimethylamine, monoethylamine, diethylamine, triethylamine, mono-n-propylamine, di-n-propylamine, tri-n-propylamine, mono-isopropylamine, and di-isopropylamine.

8. The method according to any one of claims 1 to 7,

   wherein the fluorinated ether is represented by General Formula: $R^4$-O-$R^5$,
   where $R^4$ and $R^5$ are the same as or different from each other, and represent $C_nH_mF_l$ where n is an integer of 1 to 20, m + l = 2n + 1, and m and l are an integer of 0 or more.

9. The method according to any one of claims 1 to 8,
   wherein the fluorinated ether is at least one selected from the group consisting of 1,1,1,2,2,3,3,4,4-nonafluoro-4-methoxybutane (HFE-7100), 1,1,1,2,2,3,3,4,4-nonafluoro-4-ethoxybutane (HFE-7200), and 1,1,1,2,2,3,4,5,5,5-decafluoro-3-methoxy-4-(trifluoromethyl)pentane (HFE-7300).

10. A composition, comprising:

    difluoromethane (HFC-32);
    a component X; and
    a substance A,
    wherein a total concentration of the three components is 99.5% by mass or more relative to a total of the composition,
    the component X is at least one selected from the group consisting of pentafluoroethane (HFC-125), 1,1,1-trifluoroethane (HFC-143a), and dichlorodifluoromethane (CFC-12),
    the substance A is at least one of an amine and a fluorinated ether, and
    a content of the component X is more than 0% by mass and 0.4% by mass or less relative to the total of the composition, and a content of the substance A is more than 0% by mass and 0.1% by mass or less relative to the total of the composition.

11. A composition, comprising:

    difluoromethane (HFC-32); and
    a substance A,
    wherein a total concentration of the two components is 99.5% by mass or more relative to a total of the composition,
    the substance A is at least one of an amine and a fluorinated ether, and
    a content of the substance A is more than 0% by mass and 0.1% by mass or less relative to a total of the composition.

12. A composition, comprising:

    difluoromethane (HFC-32); and
    a component X,
    wherein a total concentration of the two components is 99.5% by mass or more relative to a total of the composition,
    the component X is at least one selected from the group consisting of pentafluoroethane (HFC-125), 1,1,1-trifluoroethane (HFC-143a), and dichlorodifluoromethane (CFC-12), and
    a content of the component X is more than 0% by mass and 0.4% by mass or less relative to a total of the composition.

Fig. 1

## INTERNATIONAL SEARCH REPORT

| International application No. |
|---|
| **PCT/JP2024/022124** |

**A. CLASSIFICATION OF SUBJECT MATTER**

*C07C 17/386*(2006.01)i; *C07C 19/08*(2006.01)i; *C09K 5/04*(2006.01)i
FI: C07C17/386; C07C19/08; C09K5/04 E

According to International Patent Classification (IPC) or to both national classification and IPC

**B. FIELDS SEARCHED**

Minimum documentation searched (classification system followed by classification symbols)

C07C17/00; C07C19/00; C09K5/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2007-091762 A (E.I. DU PONT DE NEMOURS AND COMPANY) 12 April 2007 (2007-04-12) claim 1 | 1-9 |
| A | JP 2004-532206 A (HONEYWELL INTERNATIONAL, INC.) 21 October 2004 (2004-10-21) claims 1-19 | 1-9 |
| A | JP 2002-509899 A (ALLIED-SIGNAL INCORPORATED) 02 April 2002 (2002-04-02) claims 1-20 | 1-9 |
| A | JP 2001-513520 A (E.I. DU PONT DE NEMOURS AND COMPANY) 04 September 2001 (2001-09-04) claims 1-11 | 1-9 |
| A | JP 7-291878 A (SHOWA DENKO K.K.) claims 1-3 | 1-9 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **09 September 2024** | **17 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2024/022124** |

**C.    DOCUMENTS CONSIDERED TO BE RELEVANT**

| Category* | Citation of document, with indication, where appropriate, of the relevant passages | Relevant to claim No. |
| --- | --- | --- |
| X | JP 9-151370 A (ASAHI GLASS COMPANY, LIMITED) 10 June 1997 (1997-06-10) claims 1-3, paragraph [0042] | 10-12 |

Form PCT/ISA/210 (second sheet) (July 2022)

## INTERNATIONAL SEARCH REPORT
### Information on patent family members

International application No.

**PCT/JP2024/022124**

| Patent document cited in search report | | | Publication date (day/month/year) | Patent family member(s) | | | Publication date (day/month/year) |
|---|---|---|---|---|---|---|---|
| JP | 2007-091762 | A | 12 April 2007 | US claim 1 | 6156161 | A | |
| | | | | WO | 1997/003936 | A1 | |
| | | | | EP | 1029840 | A1 | |
| | | | | CN | 1196037 | A | |
| | | | | KR | 10-1999-0028943 | A | |
| JP | 2004-532206 | A | 21 October 2004 | US claims 1-19 | 2003/0010618 | A1 | |
| | | | | WO | 2002/076915 | A2 | |
| | | | | EP | 1370507 | A2 | |
| | | | | CN | 1511129 | A | |
| | | | | KR | 10-2004-0002879 | A | |
| JP | 2002-509899 | A | 02 April 2002 | US claims 1-18 | 6488817 | B1 | |
| | | | | WO | 1999/050208 | A1 | |
| | | | | EP | 1066231 | A1 | |
| | | | | KR | 10-0598890 | B1 | |
| JP | 2001-513520 | A | 04 September 2001 | US claims 1-11 | 2003/0116422 | A1 | |
| | | | | WO | 1999/007660 | A1 | |
| | | | | EP | 1003699 | A1 | |
| JP | 7-291878 | A | | (Family: none) | | | |
| JP | 9-151370 | A | 10 June 1997 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

*   JP 2007091762 A

**Non-patent literature cited in the description**

*   *CHEMICAL ABSTRACTS*, 74-89-5
*   *CHEMICAL ABSTRACTS*, 124-40-3
*   *CHEMICAL ABSTRACTS*, 75-50-3
*   *CHEMICAL ABSTRACTS*, 75-04-7
*   *CHEMICAL ABSTRACTS*, 109-89-7
*   *CHEMICAL ABSTRACTS*, 121-44-8
*   *CHEMICAL ABSTRACTS*, 107-10-8
*   *CHEMICAL ABSTRACTS*, 142-84-7

*   *CHEMICAL ABSTRACTS*, 102-69-2
*   *CHEMICAL ABSTRACTS*, 75-31-0
*   *CHEMICAL ABSTRACTS*, 108-18-9
*   *CHEMICAL ABSTRACTS*, 163702-07-6
*   *CHEMICAL ABSTRACTS*, 163702-08-7
*   *CHEMICAL ABSTRACTS*, 163702-05-4
*   *CHEMICAL ABSTRACTS*, 163702-06-5
*   *CHEMICAL ABSTRACTS*, 132182-92-4